# EUROPEAN PATENT APPLICATION

(11) **EP 4 744 644 A1**
(43) Date of publication of application: **20.05.2026**
(21) Application number: 24383242.5
(22) Date of filing: 15.11.2024
(51) Int. Cl.: A61K 8/14, A61K 8/64, A61K 8/67, A61Q 19/00, A61Q 19/08

(54) **COSMETIC PREPARATION FOR REJUVENATING THE SKIN**

(71) Applicant: Prima-Derm, S.L., 08850 Gavà (ES)
(72) Inventor: SEGOVIA LASERNA, María del Rosario, 08850 GAVÀ (ES); BERMÚDEZ HERROJO, Ignacio, 08850 GAVÀ (ES); JULIÀ MÖLLER, Ricard, 08017 BARCELONA (ES)
(74) Representative: Sugrañes, S.L.P.

(57) **Abstract**

The present disclosure relates to a cosmetic composition for rejuvenating the skin comprising between 0,01% and 0,5% by weight with respect to the total weight of the composition of a precursor of retinoic acid selected from the group consisting of retinaldehyde, retinol, retinyl retinoate, retinyl esters and combinations thereof, between 0,0001% and 0,01% by weight with respect to the total weight of the composition of an anti-aging or antioxidant peptide, and between 0,0000005% and 0,000005% by weight with respect to the total weight of the composition of a growth factor, wherein the precursor of retinoic acid is encapsulated by an encapsulating agent comprising phospholipids. The present disclosure also relates to a method for rejuvenating the skin, comprising the topical application of the cosmetic composition disclosed herein and to a use of the cosmetic composition disclosed herein for rejuvenating the skin, by means of its topical application.

## Description

### TECHNICAL FIELD

The present disclosure relates to a cosmetic composition for rejuvenating the skin. The present disclosure also relates to a method for rejuvenating the skin and to uses of the compositions herein disclosed.

### BACKGROUND ART

The skin forms the outer layer of the human being and is one of the most important organs. It has a surface area of about 2 m² and weighs 4-5 kg (6% of the total body weight). It consists of three morphologically differentiated layers (epidermis, dermis, hypodermis) that perform different functions while interrelating with each other. Many of its physiological functions are determined by its stratified structure. The epidermis consists mainly of keratinocytes that undergo a process of differentiation into corneocytes during their migration from the basal layers to the outermost layer, the stratum corneum. These corneocytes together with the surrounding lipids provide the physical and chemical barrier function of the skin. The epidermis also contains melanocytes that synthesize melanin and transfer it to keratinocytes that migrate to the upper layers.

The dermis lies below the epidermis and supports the epidermis by interacting with it through the dermal-epidermal junction. The main cell type in the dermis is fibroblasts, which are responsible for the synthesis of extracellular matrix components such as collagen, elastin and glycosaminoglycans.

The skin plays an important role as a barrier that separates us from the external environment by regulating and preventing, among other things, water loss and the entry of microorganisms and xenobiotics. It relates us to the external environment and protects us. But it also has a great social impact since its appearance influences the social behavior of most individuals. Skin aging is a continuous process where the functional homeostasis of the skin is altered and different changes in its appearance appear.

Skin aging is a multifactorial process induced by both intrinsic and extrinsic causes. It results in phenotypic changes, as well as in the structure and functions of the components of the extracellular matrix such as collagen, elastin and glycosaminoglycans involved in providing strength, elasticity and hydration to the skin.

The morphological changes produced by aging are characterized by a thinner epidermis and dermis with a lower number of keratinocytes and fibroblasts. Thinning of the epidermis increases the aged appearance of the skin with the presence of fine wrinkles while compromising barrier function and increasing water loss. The thinning of the dermis due to a decrease in collagen and elastin fibers increases its fragility and results in a loss of firmness.

Retinoids are natural or synthetic molecules that have structural similarities to vitamin A (retinol), one of the essential nutrients for our body. Vitamin A plays a key role in several biological functions, including: vision, growth and development, reproduction, proper functioning of the immune system, and contributes to the health of the skin and mucous membranes, helping to maintain their integrity and barrier function.

The human body does not produce vitamin A naturally, so it must be acquired through the diet in the form of preformed vitamin A, which is found in foods of animal origin such as liver, fish, eggs and dairy products, or in the form of provitamin A (carotenoids), present in orange, yellow and dark green fruits and vegetables, such as carrots, sweet potatoes, spinach and broccoli. A daily intake of 0.7 mg of vitamin A is recommended for women and 0.9 mg for adult men, while the tolerable upper intake level (UL) is 3 mg per day. Vitamin A deficiency is the leading cause of preventable blindness in children worldwide. In pregnant women, vitamin A deficiency causes night blindness and can contribute to maternal mortality. In addition, it can also damage the immune system, cause thyroid disorders and skin disorders. Conversely, excessive consumption can lead to hypervitaminosis, which can be highly toxic, especially before and during pregnancy due to possible teratogenic effects.

In relation to the skin, the effect of vitamin A attracted the attention of dermatologists because vitamin A deficiency in humans was found to produce diseases related to skin keratinization, such as acne, psoriasis, ichthyosis or lichen planus, among others. The following are some historical milestones in the use of retinoids in medicine for the management of skin diseases:
- In 1943, vitamin A began to be administered orally for the treatment of acne. The acid form of this vitamin, retinoic acid, also began to be synthesized and administered orally for the treatment of keratinization disorders, which resulted in a revolution in the management of these diseases.
- In 1969, topical retinoic acid was used in acne, with good results.
- In 1972, other synthetic derivatives with vitamin A analogous activity began to be investigated, seeking more specificity and fewer adverse effects: acitretin, tazarotene, adapalene and bexarotene.

Numerous vitamin A analogs are currently available in the art, although in most cases their use is limited to the medical setting. Only retinol, retinal and retinyl esters can be used for cosmetic purposes.

When applied topically to the skin, retinoids can penetrate the epidermis and dermis and reach keratinocytes and fibroblasts, where they coexist and exert different functions in multiple cellular compartments. First, retinoid storage is mostly regulated in the cytoplasm of keratinocytes, where retinoids are transformed to retinyl esters by esterification reactions controlled by lecithin retinol acyltransferase (LRAT) and acyl-CoA retinol acyltransferase (ARAT). These stored retinyl esters can subsequently be converted back to retinol by esterases. Also in the cytoplasm, retinol undergoes a two-step conversion process to retinoic acid. In the first step, the enzyme alcohol dehydrogenase (ADH) catalyzes the conversion of retinol to retinaldehyde, while, in the second step, retinaldehyde dehydrogenase (RALDH) converts retinaldehyde to retinoic acid.

Retinoic acid binds the intracellular retinoic acid binding protein (CRABPII). The retinoic acid-CRABPII complex can translocate to the cell nucleus, thus ultimately exerting its effect as a ligand for retinoic acid receptors and retinoid X receptors (RAR and RXR). When these receptors are bound to retinoic acid (RA-RAR/RXR), they interact with large protein complexes that control gene transcription. These genes may encode proteins involved in the regulation of cell growth, differentiation and the synthesis of extracellular matrix components, among other processes.

Retinoids are among the most studied ingredients in skin care to combat aging and improve skin's appearance. Their effects are widely demonstrated at both the epidermal and dermal levels, exerting their influence mainly on three key categories of skin cells: epidermal keratinocytes, dermal fibroblasts and endothelial cells. Its main beneficial effects are detailed below:
1. Topical application of retinoids has a significant impact on increasing the thickness of the epidermis. This is due to the significant increase in the expression of the c-Jun protein, which is one of the main components of the AP-1 transcription factor, which is of utmost importance in the proliferation of epidermal keratinocytes.
2. Stimulates the proliferation of endothelial cells and blood vessels in the dermis. This increased dermal vascularization promotes better circulation in the skin, creating a more conducive environment for epidermal and dermal homeostasis.
3. Triggers activation of the TGF-β/CTGF pathway, a key regulator of extracellular matrix balance, resulting in increased expression of type I collagen, the major structural protein of the skin. In addition to the increase in type I collagen, the expression of fibronectin and tropoelastin is significantly enhanced.
4. Retinoids regulate melanogenesis by reducing the activity of tyrosinase, a key enzyme in melanin synthesis, decreasing melanin production and its transfer to keratinocytes. In addition, they accelerate cell renewal and help exfoliate pigmented cells. They also have anti-inflammatory properties, making it effective in the treatment of post-inflammatory hyperpigmentation.
5. Retinoids also exhibit anti-acne effects, due to their anti-inflammatory effects that include inhibition of bacteria-induced proinflammatory pathways, reduction in cytokine and nitric oxide release. In addition, some compounds such as retinal and retinoic acid themselves exhibit bactericidal activity against the microorganism *Propionibacterium acnes.*

For instance, EP2649986 A2 discloses a cosmetic composition for rejuvenating the skin characterized in that it comprises a precursor of retinoic acid from the group made up of retinaldehyde, retinol, retinyl retinoate and retinyl esters, and melatonin.

US2011262373 A1 discloses a personalized pharmaceutical composition for skin rejuvenation comprising a first group of common active ingredients comprising retinoic acid, one or more anti-inflammatory agents, one or more depigmenting agents, one or more antioxidants, and one or more vitamins; and a second group of variable active ingredients, wherein at least one anti-inflammatory agent is indomethacin.

The European Commission's Scientific Committee on Consumer Safety (SCCS) published in October 2022 a final opinion limiting the use of retinol and retinol esters in cosmetic products marketed in the European Union as of June 2024.

This Opinion was developed because of the growing concern that a percentage of the population was being exposed to doses of Vitamin A in excess of the recommended daily allowances for their overall intake through diet, food supplements and cosmetic products.

The SCCS is of the opinion that vitamin A in cosmetics at concentrations of 0.05% retinol equivalent (RE) in body lotions and 0.3% RE in other leave-on and rinse-off products is safe. Therefore, use is limited to 0.3% retinol and retinol esters in non-rinse-off cosmetic products. This limitation does not affect retinal or other molecules not specifically addressed in the SCCS Opinion.

Retinaldehyde or retinal is a molecule that requires one step less of transformation to become retinoic acid, therefore its potency is higher than retinol and the side effects could be greater, although there is a current of opinion that recommends retinal for more reactive skins since a dose about ten times lower than retinol is needed to obtain a similar effect.

The efficacy of a cosmetic product depends largely on the characteristics of its formula and the concentration of the active ingredients it contains, as well as on the capacity of these active ingredients to reach the biological targets on which they must act. It is for this reason that delivery systems are today one of the greatest challenges in the cosmetic industry, as they make it possible to improve product efficacy by increasing the penetration of active ingredients into the skin. As is well known, human skin acts as a barrier against the permeation of exogenous molecules. The outermost layer of the skin, the stratum corneum, is a complex structure formed by keratinized cells that are surrounded by a lipid matrix of varying composition. The lipids present in this matrix are ceramides, cholesterol and fatty acids.

One of the most studied delivery systems to date in the world of cosmetics is liposomes, first described in 1964 by Bangham et al. A liposome is a spherical structure composed mainly of a lipid bilayer, surrounding an aqueous core and usually with a particle size between 20 nm and 5 µm. These liposomes are obtained from a wide variety of lipids, the most used being phospholipids. A phospholipid has a polar or hydrophilic group at the head and two hydrophobic hydrocarbon tails. When such compounds are in the presence of excess aqueous solution, they organize themselves in a way that minimizes interactions between water and hydrocarbon chains. This system triggers the spontaneous formation of lipid lamellae in the form of spherical bilayers. The lamellar structure of liposomes allows them to encapsulate hydrophilic molecules in their aqueous core and hydrophobic molecules inside their lipid bilayer.

For instance, US12059486 discloses a method to prepare a tocopherol phosphate mixture liposomal composition comprising, among other features, the mixture of a lipophilic ingredient that may be a retinoid, that is encapsulated in the liposome.

However, there remains in the art a need for cosmetic compositions encapsulating retinoids that are stable, have increased skin penetration, show a higher anti-wrinkle efficacy, a convenient luminosity effect and/or provide with good values of elasticity/firmness in the skin after application.

### SUMMARY OF THE DISCLOSURE

One aspect of the present disclosure provides a cosmetic preparation for rejuvenating the skin comprising between 0,01% and 0,5% by weight with respect to the total weight of the composition of a precursor of retinoic acid selected from the group consisting of retinaldehyde, retinol, retinyl retinoate, retinyl esters and combinations thereof, between 0,0001% and 0,01% by weight with respect to the total weight of the composition of an anti-aging or antioxidant peptide, and between 0,0000005% and 0,000005% by weight with respect to the total weight of the composition of a growth factor, wherein the precursor of retinoic acid is encapsulated by an encapsulating agent comprising phospholipids.

A second aspect of the present disclosure provides a method for rejuvenating the skin, characterized in that it comprises the topical application of the cosmetic composition as disclosed herein.

A third aspect of the present disclosure relates to the use of the cosmetic composition as disclosed herein for rejuvenating the skin, by means of its topical application.

Other features of the invention are disclosed in the detailed description of the invention.

### BRIEF DESCRIPTION OF THE FIGURES

The foregoing and other advantages and features will be more fully understood from the following detailed description of an embodiment with reference to the accompanying drawings, to be taken in an illustrative and non-limitative manner, in which:
FIG. 1 accounts for two graphs illustrating the distribution of size particle of encapsulated retinal in three different samples (graph above) and the Z-potential obtained of the different samples of encapsulated retinal (graph below).
FIG. 2 is a comparative graph showing retinal stability in different compositions, as determined by reverse-HPLC with 350 nm wavelength detection at room temperature.
FIG. 3 depicts three HPLC chromatograms for Acetyl tetrapetide-2 (FIG. 3A), for the cosmetic composition according to the disclosure with retinal at 0,1 % w/w just after preparation (time 0) (FIG. 3B), and for the cosmetic composition according to the disclosure with retinal at 0,1% w/w after being kept for three months at room temperature (FIG. 3C).
FIG. 4 is a graph depicting the amount of retinal absorbed in synthetic skin in the composition according to the present disclosure in two retinal concentrations and the amount of retinal absorbed in comparative compositions.
FIG. 5 is a graph showing the penetration of the composition according to the disclosure an equivalent composition without retinal encapsulation according to the *in vivo tape stripping* method.

### DESCRIPTION OF THE DISCLOSURE

The foregoing and other advantages and features will be more fully understood from the following detailed description of the invention. The examples disclosed herein are to be taken in an illustrative and not limitative way.

Unless otherwise indicated, all percentages relating to the content of a component or to a collection of components of the composition of the present invention refer to the weight percentage with respect to the total weight of the composition.

In the context of the present disclosure, the formulations "according to the disclosure", "preparation according to the disclosure", etc. always refer to the preparations, processes and uses according to the disclosure, i.e. also to preparations in which the uses according to the disclosure are realized as well as preparations with which the inventive method is realized.

Unless the context clearly requires otherwise, throughout the description and the claims, the words "comprise", "comprising", and the like are to be construed in an inclusive sense as opposed to an exclusive or exhaustive sense; that is to say, in the sense of "including, but not limited to".

The cosmetic composition is as defined in the present disclosure, that is, a cosmetic composition for rejuvenating the skin comprising between 0,01% and 0,5% by weight with respect to the total weight of the composition of a precursor of retinoic acid selected from the group consisting of retinaldehyde, retinol, retinyl retinoate, retinyl esters and combinations thereof, between 0,0001% and 0,01% by weight with respect to the total weight of the composition of an anti-aging or antioxidant peptide, and between 0,0000005% and 0,000005% by weight with respect to the total weight of the composition of a growth factor, wherein the precursor of retinoic acid is encapsulated by an encapsulating agent comprising phospholipids.

Preferably, the precursor of retinoic acid is retinaldehyde. Retinaldehyde and retinal are equivalent terms. Therefore, in the present disclosure, retinal and retinaldehyde may be equivalently used and refer to the compound having a C₂₀H₂₈O as general formula, a molecular weight of 284,214 g/mol and a CAS number 311338-94-0.

An anti-aging peptide or antioxidant peptide is a small chain of aminoacids, a peptide, that plays a role in counteracting the biological effects of aging, often by neutralizing free radicals or protecting cells from oxidative stress. These peptides work in various ways to support skin health, reduce wrinkles, promote cellular regeneration, and combat the damage caused by environmental stressors like UV radiation, pollution, and inflammation. In particular, anti-aging peptides generally focus on improving the appearance and health of the skin, enhancing cellular function, and promoting tissue repair while antioxidant peptides protect cells from damage caused by free radicals-unstable molecules that can cause oxidative stress, leading to cell damage and aging, neutralizing free radicals or modulating antioxidant pathways within the body to reduce oxidative damage.

Preferably, the anti-aging or antioxidant peptide is selected from the group consisting of carnosine, copper tripeptide-1, pentapeptide-18, palmitoyl tetrapeptide-7, palmitoyl pentapeptide-4, nonapeptide-1, hexapeptide-2, acetyl hexapeptide-8, acetyl octapeptide-3, acetyl hexapeptide-1 o acetyl tetrapeptide-2, and combinations thereof.

Even more preferably, the anti-aging or antioxidant peptide is acetyl tetrapeptide-2.

Acetyl tetrapeptide-2 has primary antioxidant activity and antioxidant amplifier since it inhibits the aryl receptor (AHR) and stimulates the NRF2-like Nuclear Factor (erythroid-derived factor 2) that activates the detoxifying and endogenous antioxidant systems HMOX-1, GSS, GPX1, GSTP1 and TRX1.

In addition to its antioxidant and anti-pollution effect proven by ex vivo experiments with human skin explants, an in vivo study on 20 Asian women proved its illuminating effect by decreasing the overall melanin values of the face.

Preferably, the cosmetic composition as disclosed herein comprises an anti-aging or antioxidant peptide in an amount of between 0,0005% and 0,005% by weight with respect to the total weight of the composition, more preferably between 0,00075 and 0,0025, more preferably about 0,001%.

A growth factor is a substance, usually a protein or a steroid, that stimulates cell growth proliferation, healing, and differentiation. Growth factors play a crucial role in regulating a variety of cellular processes, including embryonic development, tissue repair, immune responses, and the maintenance of normal cellular functions. They act as signaling molecules that bind to specific receptors on the surface of cells, triggering intracellular pathways that promote various cellular activities.

Preferably, the growth factor in the cosmetic composition as disclosed herein is selected from the group consisting of epidermal growth factor, also known as Oligopeptide-1, insulin-like growth factor 1, transforming growth factor beta-2, Nicotiana Benthamiana Hexapeptide-40 sh-Polypeptide-76, sh-Polypeptide-1, sh-Polypeptide-9, sh-Polypeptide-11, Oligopeptide-4, Oligopeptide-2, and combinations thereof.

More preferably, the growth factor in the cosmetic composition as disclosed herein is Oligopeptide-1, epidermal growth factor, and/or combinations thereof.

The epidermal growth factor (EGF) as a cosmetic ingredient may be preferably obtained using *Nicothiana Benthamiana* as plant biofactory.

This growth factor obtained by this technology has all the glycosylations and a tertiary structure similar to endogenous growth factor, which increases its efficacy.

EGF is a widely known therapeutic polypeptide that exerts a mitogenic effect by binding to its membrane receptor EGFR, regulating cell growth, proliferation and differentiation among other biological aspects. The EGFR receptor is found in most epithelial tissues, fibroblasts and endothelial tissues and is therefore key in maintaining the integrity of these tissues.

EGF has been widely used therapeutically in the treatment of chronic wounds, the prevention of scarring and the treatment of skin dermatitis caused by radiotherapy or chemotherapy.

At the cosmetic level its interest is determined by its capacity to stimulate fibroblasts and increase the synthesis of collagen and hyaluronic acid. In vivo studies conducted with EGF on 20 Caucasian female volunteers demonstrated an anti-aging effect in the measurement of different skin parameters. Improvements in skin roughness and reduction of wrinkles were observed.

Preferably, the cosmetic composition disclosed herein comprises the growth factor in an amount of between 0,000001% and 0,000003% by weight with respect to the total weight of the composition, more preferably between 0,0000015 and 0,0000025, even more preferably about 0,0000021%.

In the cosmetic composition as disclosed herein, the phospholipids are preferably derived from lecithin.

Lecithin comprises mixtures of glycerophospholipids including phosphatidylcholine, phosphatidylethanolamine, phosphatidylinositol, phosphatidylserine, and phosphatidic acid. The phospholipids according to the present disclosure may be selected from the group consisting of phosphatidylcholine, phosphatidylethanolamine, phosphatidylinositol, phosphatidylserine, phosphatidic acid, and combinations thereof.

Preferably, in the cosmetic composition according to the present disclosure the phospholipids are in an amount of between 0,1% and 2% by weight with respect to the total amount of the composition. Preferably, the amount is between 0,3% and 1%, more preferably between 0,4% and 0,8%, even more preferably about 0,6%.

The encapsulating agent in the cosmetic composition according to the present invention may preferably be a liposome. The liposome may preferably comprise one lipidic bilayer, but other liposomes comprising two or more lipidic bilayers are also envisaged.

The cosmetic composition according to the present disclosure may further comprise an emollient.

An emollient in cosmetics is a substance that softens and smooths the skin by forming a protective barrier on the surface. It helps to maintain moisture in the skin, preventing dryness and irritation. Emollients work by filling in the gaps between skin cells, making the skin feel softer, more hydrated, and less rough or flaky.

The emollients in the cosmetic composition according to the present disclosure may preferably be selected from the group consisting of squalene, sweet almond oil and combinations thereof.

The cosmetic composition according to the present disclosure may also comprise water and an aliphatic polyalcohol, the aliphatic polyalcohol being preferably selected from the group consisting of glycerol, butylene glycol, caprylyl glycol and combinations thereof.

As described herein, the present disclosure also relates to a method for rejuvenating the skin, the method comprising topical application of the cosmetic composition as disclosed herein.

As described herein, the present disclosure also relates to the use of the cosmetic composition according to the present disclosure for rejuvenating the skin by means of topical application.

The cosmetic composition described herein with the proportions of the different components described and encapsulated by an encapsulating agent is found to enhance the effect of retinaldehyde, allows a stable formulation, the skin penetration of the cosmetic composition is convenient, the anti-wrinkle efficacy is high, provides the skin with a convenient luminosity effect and with good values of elasticity/firmness.

### EXAMPLES

### Example 1: Retinal encapsulation

Retinal is a highly unstable molecule to light, heat, oxygen and free radicals. In addition, it is a fat-soluble ingredient, which makes it difficult to incorporate into aqueous formulations. In order to improve its solubility, stability and in turn improve its absorption into the skin, in an example of the present invention, pure retinal was encapsulated in liposomes using an optimized lipid matrix composed of phosphatidylcholine and other lipids from non-GMO soybeans.

The encapsulated retinal was analyzed by Dynamic Light Scattering, a technique allowing to obtain the particle size and the Z potential of vesicles, this parameter being a measure of the magnitude of electrostatic repulsion or attraction between particles, a fundamental parameter to predict the stability of a formulation composed of liposomes.

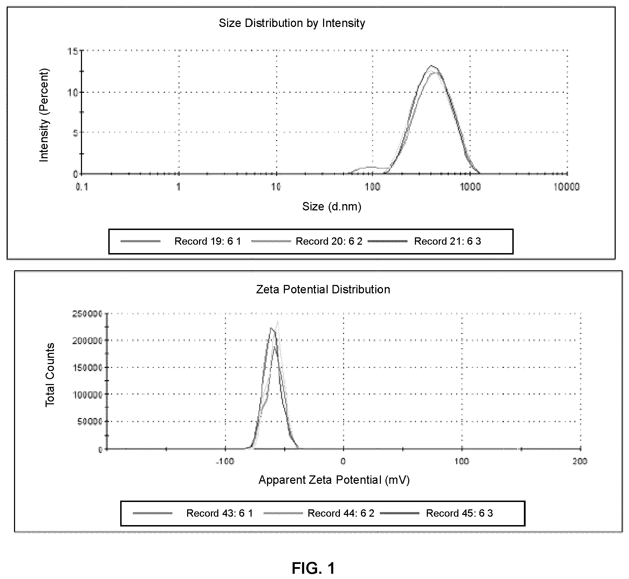

The liposomes encapsulating retinal showed a Z potential lower than -60 mV and an approximate size of 350 nm of diameter, as shown in FIG. 1.

### Example 2: Formulation of the cosmetic composition

The cosmetic composition according to the disclosure was prepared from the obtained encapsulated retinal as described above. Two different cosmetic compositions, having 0.1% or 0.2% of retinal (by weight with respect to the total amount of the compositions) were prepared.

Two other active ingredients were added, so as to complement and potentiate the effects provided by retinal:
- Epidermal Growth Factor (EGF), also known as Oligopeptide-1, was added so that EGF was in an amount of 0,0000021% by weight with respect to the total amount of the cosmetic composition. EGF commercial name was Epitensive^{®} from Lipotrue SL.
- Acetyl tetrapeptide-2 was added so that Acetyltetrapeptide-2 was in an amount of 0,001% by weight with respect to the total amount of the cosmetic composition. Acetyl tetrapeptide-2 commercial name was Neoclair Pro^{®} from Lipotrue SL.

### Example 3: Stability studies

Due to its five conjugated double bonds, retinal is a highly unstable molecule to light, heat, oxygen and free radicals, prone to addition, oxidation, polymerization, cleavage and/or dehydration reactions. For this reason, in addition to the study of the stability of the formula, in this case it is of vital importance to study the stability of the active ingredient in contact with the rest of the ingredients. This study was carried out by HPLC quantification of the encapsulated retinal present in the cosmetic composition (in two concentrations, 0.2% and 0.1% by weight, called Retinal Renewal 0,1% and Retinal Renewal 0,2%) and compared as well to five other retinal formulations commercially available. The features of these five products are summarized below (Table 1):

**Table 1: Comparative compositions comprising retinal in an encapsulated or non-encapsulated formulation.**

| Comparative composition | Manufacturer | Retinal concentration (% w/w) | Retinal encapsulation |
|---|---|---|---|
| 1 | A | 0.1 | Not disclosed |
| 2 | A | 0.1 | Liposomes |
| 3 | A | 0.05 | Liposomes |
| 4 | B | 0.06 | Encapsulated |
| 5 | B | 0.2 | Encapsulated |
| 6 | C | Not disclosed | Not disclosed |

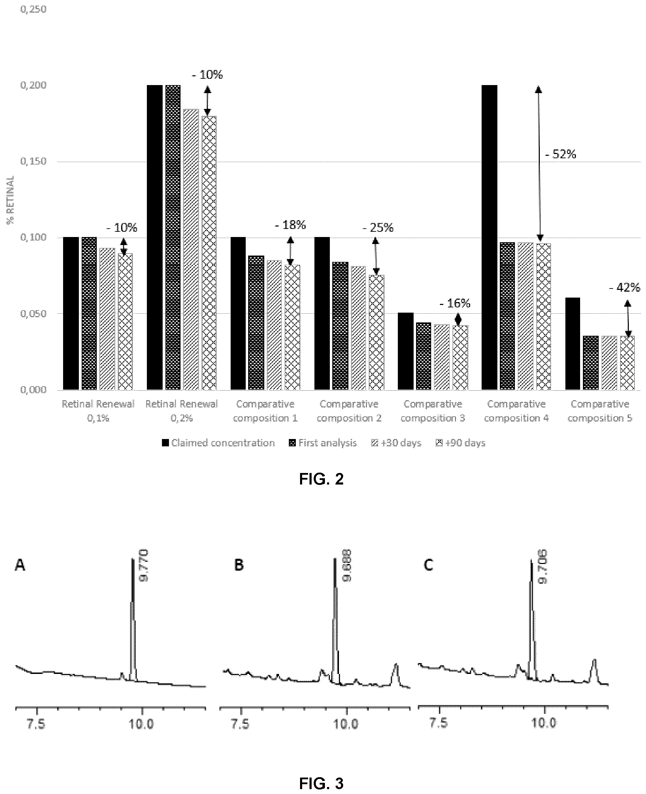

It was observed that in the first 3 months there is a degradation of less than 10% (see FIG. 2). Neither the two samples (Retinal Renewal 0,1% or Retinal Renewal 0,2%) according to the present cosmetic composition showed significant degradation. It should be noted that this degradation occurs mainly during the first month of the product's life, since during the manufacturing process the ingredient is more exposed to external conditions such as the presence of oxygen or light. Subsequently, the degree of degradation slows down and the concentration remains practically constant over time, so it can be expected that retinal will remain stable in the following 6-month and 1-year samples. Therefore, it can be concluded that the encapsulated retinal will remain in optimal condition throughout the entire life cycle of the product. In addition, the final packaging of the product will contribute to the stability of the ingredient as it is totally opaque and airless (no light or oxygen present).

The stability of the retinal in the comparative compositions (competitor products) was also analyzed. In this case it must be considered that it is not known the period of time elapsed between the production of the product by the manufacturer and the date on which the product was purchased and analyzed. In none of the products analyzed was the concentration claimed by the manufacturer found, with Comparative Composition 4 being the product with the greatest difference between the result obtained and the expected result (reduction of retinal concentration by 52%). This means that in the commercial product that should have 0.2% retinal, there is really only 0.097% in its active form. This may be due to degradation of the retinal over time or due to encapsulation, as manufacturer B stabilizes the retinal by encapsulating, forming a complex that may be so strong that half of the retinal remains in the formula in an inactive form. In the case of manufacturer A's products (Comparative Compositions 1 to 3), the deviation between the claimed concentration and the concentration obtained is much smaller, around 15%. it should be noted that at the time of the analysis the products had been recently manufactured as they had just been launched on the market. Finally, the initial concentration of the Comparative Composition 6 is unknown since it was not disclosed by the manufacturer. This product has a two-phase container, where the retinal is dissolved in an emollient phase and separated from the rest of the ingredients of the formula. The amount of retinal in the final product was analyzed by HPLC once both phases were mixed and it was determined that the concentration oscillates around 0.05%, being this variable since the container supplies different proportion of each phase according to the dosing device. As for its stability, the retinal remains stable over time as it is separated from the rest of the ingredients. It should be noted that the cosmetic, once both phases are mixed, is not stable and separates in less than 30 days at room temperature.

The stability of the Acetyl tetrapeptide-2 in contact with retinal and the other ingredients of the cosmetic composition was analyzed by HPLC. FIG. 3 shows that the retention time and the area under the curve for the peak corresponding to Acetyl tetrapeptide-2 in the control (FIG. 3A) and in the cosmetic composition at time 0 (FIG. 3B) and after three months (FIG. 3C) is equivalent. In particular, the retention times are 9,770, 9,688 and 9,706 min for A, B and C, respectively and the Area are 224583, 223045 and 222996 for A, B and C, respectively.

### Example 4: Efficacy studies

### Percutaneous absorption

Percutaneous absorption studies are widely used in the pharmaceutical, cosmetic and dermatological industries to evaluate the efficacy and safety of topical products, as well as to better understand the mechanisms of skin absorption. These studies are performed using a device called a Franz cell, which consists of two compartments separated by a membrane.

In the upper compartment the substance to be studied is placed, while in the lower compartment a receptor medium that simulates the pH, salinity and temperature conditions under the skin is placed. The membrane used in the Franz cell is key in simulating the skin barrier and measuring the absorption of the substance through it. This membrane can be of animal or artificial origin, made of different materials, such as cellulose or synthetic polymers. In our case we used a commercial synthetic membrane called Strat-M^{®}, which has a multilayer structure that mimics the composition and characteristics of the skin. This membrane is very useful in basic and applied research studies, as it reduces the need to use human or animal skin in experiments, which has ethical and regulatory advantages. In addition, it has been widely demonstrated to provide consistent, reproducible results with a high correlation to those obtained in human skin.

During the study, the integrity of the skin barrier and the tightness of the experimental model are verified for each diffusion cell before the application of the cosmetic products by measuring the transepidermal water loss (TEWL). The sample to be analyzed is then applied to the donor compartment and left in contact with the synthetic skin for 24 hours. After the contact time has elapsed, the three parts of the cell (donor, membrane and receptor) are sampled separately. Finally, the percutaneous absorption of the active ingredient is studied quantitatively by HPLC. Each sample was analyzed in triplicate and on two different days to avoid human error and environmental effects. The average of these measurements was calculated, their standard deviation and the statistical significance of the data was analyzed by Student's t-test.

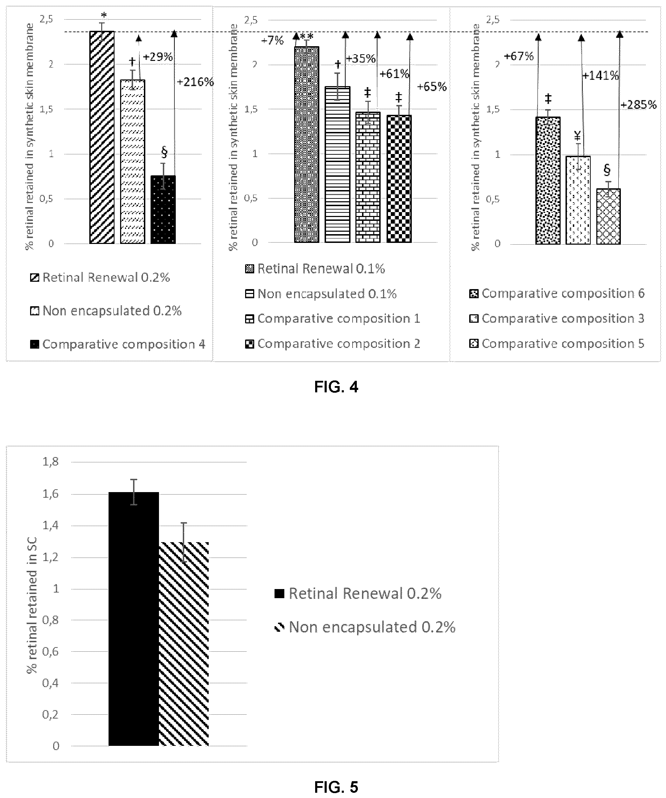

FIG. 4 shows the results of percutaneous absorption of retinal in the different products tested. The efficacy of encapsulation was evaluated by studying the cosmetic composition according to the disclosure in its two concentrations (Retinal Renewal 0,1% and Retinal Renewal 0,2%) and its analogues without encapsulation (Comparative compositions 1 to 6). It was found that for both concentrations, encapsulating the retinal increases its absorption into the skin by 29% and 35%, respectively (first and second bar of FIG. 4A and FIG. 4B). The compositions according to the disclosure were also tested and compared with several competitor products, proving that the encapsulation disclosed herein improves retinal penetration in all cases. It is worth noting the large differences found with the main competitors of the Applicants, which also include encapsulated retinal in their formulas, +216% compared to Comparative composition 4 and +65% compared to Comparative composition 2 (see FIG. 4A and FIG. 4B). Significant differences were also found with respect to Comparative compositions 1 (61%), 3 (141%), 5 (285%) and 6 (67%).

It is worth noting that even the non encapsulated form of the retinal as disclosed in the invention showed better results than any of the encapsulated and other comparative compositions 1 to 6.

### In vivo tape stripping

In order to validate the previously *in vitro* percutaneous absorption results, an *in vivo* study known as tape stripping was performed. This is a technique that involves the application of a special adhesive tape on the skin, followed by its removal to remove superficial layers to quantify the amount of active ingredient that has been retained in the stratum corneum of the skin.

To do this, the area of the volunteer's forearm is first cleaned and dried to ensure that there are no contaminants that could affect the results. A standardized amount of product is then applied to the area, massaged to facilitate absorption and allowed to stand for two hours. After this contact time, the area is cleaned again with abundant water. To remove the most superficial layer of the skin, medical adhesive tape is used, which is pressed firmly on the skin to ensure good adherence and is quickly removed, taking with it a layer of stratum corneum cells. This process is repeated twenty times at the same site to remove successive layers of skin. Finally, the removed strips are treated with an extractant solution, which is then analyzed to quantify the amount of substance that has penetrated the skin by HPLC.

This study was performed with the composition defined herein having a retinal concentration of 0.2% w/w and an equivalent formula without retinal encapsulation. The results obtained (see FIG. 5) are in agreement with those obtained in the in vitro study, showing that the encapsulated retinal penetrates the skin 24% more than the non-encapsulated one.

### Example 5: Anti-wrinkle efficacy

A single-center open-label study with dermatological and instrumental evaluation was performed for 28 days with 30 volunteers of both genders, men and women, having ages comprised between 30 and 70, having normal skin and instructed not to use any cosmetic products in the area to be evaluated that might alter the results of the trial in a week prior to the study.

The anti-wrinkle efficacy was quantitatively evaluated, by means of instrumental measurements of the skin, for which the Visioface 1000D^{®} equipment was used, taking measurements on the volunteers before, after 14 days and after 28 days of use of the tested product.

The measurements were carried out on the application area (face), verifying the volume, area and depth of the chosen wrinkle.

Measures were taken before the application of the product (T0), after 14 days (T14D) and after 28 days (T28D) of regular use. The study was carried out in a heated room with an ambient temperature of 20 ± 2°C and a relative humidity of 45 ± 15%.

The efficacy of the product is confirmed if positive results are obtained in more than 50% of the subjects.

The results are summarized in the following table (Table 2):

**Table 2: Mean results obtained regarding the measurements of depth, width and volume achieved after comparing the measures performed after the application of the product (T0), after 14 days (T14D) and after 23 days of the use of the product (T28D).**

| | T0 | T14D | T28D | T0-T14D | T0-T28D |
|---|---|---|---|---|---|
| VOLUME | 133806,800 | 98354,000 | 68908,667 | -30% | -52% |
| AREA | 10895,967 | 7573,500 | 5636,033 | -32% | -51% |
| DEPTH | 11,233 | 11,667 | 10,900 | 4% | -2% |
| % AREA | 1,411 | 0,974 | 0,726 | -32% | -51% |

Table 2 shows that the volume, area and depth of the wrinkles were significantly decreased after 14 and 28 days of application of the product.

### Example 6: Luminosity effect

The same single-center open-label study with dermatological and instrumental evaluation as described for Example 5 was used to measure luminosity effect. For the instrumental evaluation of the efficacy on brightness with the Chromameter CR-400, one of the readings was taken on all volunteers before the application of the product at the sites established at the baseline visit (front). Three measurements were taken at each time/area 14 days and 28 days after the start of the study.

Where the individual results were expressed in absolute values for each time and in percentage variation between times T0-T14D and T0-T28D. Measuring Principle of Chromameter CR-400.

The Skin-Colorimeter probe has a large illumination area, so that sufficient light reaches the surface of the skin for measurement, but a small enough measurement area does not ensure that the surface colour is measured and not the colour of the deeper layers of the skin.

The probe sends out white LED light, arranged circularly to illuminate the skin evenly. The emitted light is scattered in all directions, some of it travels through the layers and some is scattered away from the skin, the light reflected from the skin is measured by the probe and expressed accordingly.

The raw data of the probe is corrected with a special colour matrix to be as close as possible to normal values. Due to the unique structure of the skin and the light source of the probe, the skin cannot be measured according to standards such as DIN (the German industry standard) or others. The measured values are close to those standards but are shown as arbitrary results.

The measured skin colour is expressed as an XYZ-value (tristimulus) and can be calculated in different colour spaces.

L*a*b:
The CIELAB colour space (also known as CIE L*a*b*) is a colour space defined by the International Commission on Illumination (CIE) in 1976. It expresses colour as three values: L* for lightness from black (0) to white (100), a* from green (-) to red (+), and b* from blue (-) to yellow (+). CIELAB was designed so that the same amount of numerical change in these values corresponds to approximately the same amount of visually perceived change. It is a concept that is often used to characterise skin.

Correlation between colour space and human skin:
- L* gives information on the black-white axis, brightness/lightness, the higher the L*, the brighter the skin.
- The a* value on the green-red axis is proportional to the erythema (microcirculation/redness of the skin).

A higher a* value corresponds to a higher erythema.
- The b* value on the blue-yellow axis in the literature often describes pigmentation.

The instrumental evaluation of the efficacy on brightness was carried out by statistical analysis of the readings taken by all volunteers before applying the product (T0), after 14 days (T14D), and at 28 days after applying the product (T28D). The individual results were expressed in absolute values for each time and in percentage of variation between the times T0-T14D and T0-T28D.

Means and standard deviation were calculated.

The results are summarized in the following table (Table 3):

**Table 3: Average results of skin radiance after comparing measurements taken before product application (T0), after 14 days (T14D) and after 28 days (T28D) of product use.**

| | AVERAGE LUMINOSITY RESULTS | | | | |
|---|---|---|---|---|---|
| | Absolute values | | | % values | |
| | T0 | T14D | T28D | T0-T14D | T0-T28D |
| Average | 60,42 | 60,88 | 63,05 | 1% | 4% |
| % volunteers with global improvement | - | - | - | 53% | 100% |

The product, as seen in Table 3, has an effect between T0-T14D of the 30 volunteers of 1% and, after 28 days, the brightness level is increased by 4%.

### Example 7: Efficacy on elasticity and firmness of the skin

The same single-center open-label study with dermatological and instrumental evaluation as described for Examples 6 and 6 was used to measure efficacy of the composition herein disclosed on elasticity and firmness of the skin.

The subjects remained resting for approximately 20 minutes in an air-conditioned room, after which the test began. Both the resting room and the testing room where the test was carried out have regulated and controlled conditions of temperature (20±2°C) and humidity (45±15%).

The resting period allowed each subject to relax, and the skin to reach a balance in relation to the humidity level.

The study was carried out with a measuring device called Cutometer^{®} Dual MPA580 on facial skin, using vacuum pressure that deforms the skin mechanically after an adjustable suction time; the vacuum pressure was set to zero, making possible to visualize the changes in skin elasticity and firmness.

This measuring equipment consists of a light source and a light receiver as well as two prisms facing each other, which project the light from the transmitter to the receiver. The intensity of the light varies due to the depth of penetration into the skin. The results assess the skin's resistance to negative pressure (firmness) and its ability to return to its original position (elasticity).

Measurements were carried out before application of the product T0, after 14 days (T14D) and 28 days (T28D) of regular use at the volunteers' home.

The efficacy on skin elasticity and firmness was evaluated quantitatively using the equipment Cutometer Dual MPA 580 to perform instrumental measurements on volunteers' skin before, after 14 days and after 28 days of use of the tested product.

Measurements were taken on the area where the product was applied (face).

The results are summarized in the following table (Table 4):

**Table 4: Average results obtained for skin firmness and elasticity with measurements taken before (T0), after 14 days (T14D) and after 28 days (T28D) of use of the product.**

| | Parameter | T0 | T14D | T28D | T0-T14D | T0-T28D |
|---|---|---|---|---|---|---|
| Firmness | R0 | 0,318 | 0,251 | 0,205 | -21% | -36% |
| Gross elasticity | R2 | 40,242 | 42,603 | 47,903 | 6% | 19% |
| Neat elasticity | R5 | 41,907 | 44,292 | 51,623 | 6% | 23% |
| Elasticity over the entire surface | R7 | 32,998 | 37,924 | 48,303 | 15% | 46% |
| Average elasticity | - | 38,386 | 41,606 | 49,276 | 8% | 28% |
| % volunteers with global improvement | - | - | - | - | 40% | 100% |

Therefore, after 28 days of use, an average decrease in skin firmness and an average increase in the parameters assessed for elasticity are observed, both indicating an improvement in skin condition.

After 14 days, for volunteers with a positive reaction, the mean improvement in firmness is -21%, and the average elasticity is 8%.

After 28 days, for volunteers with a positive reaction, the average improvement in firmness is -36%, and the average elasticity is 28%.

Therefore, the results obtained from the studies with volunteers (Examples 5, 6 and 7) it can be concluded that the composition as disclosed herein is tolerated very good by the skin, has an antiwrinkle effect reducing volume, area and depth, improves skin luminosity and has a statistically significant positive effect on firmness and elasticity.

## Claims

1. Cosmetic composition for rejuvenating the skin comprising:
- between 0,01% and 0,5% by weight with respect to the total weight of the composition of a precursor of retinoic acid selected from the group consisting of retinaldehyde, retinol, retinyl retinoate, retinyl esters and combinations thereof,
- between 0,0001% and 0,01% by weight with respect to the total weight of the composition of an anti-aging or antioxidant peptide, and
- between 0,0000005% and 0,000005% by weight with respect to the total weight of the composition of a growth factor,
wherein the precursor of retinoic acid is encapsulated by an encapsulating agent comprising phospholipids.

2. The cosmetic composition according to claim 1, wherein the anti-aging or antioxidant peptide is selected from the group consisting of carnosine, copper tripeptide-1, pentapeptide-18, palmitoyl tetrapeptide-7, palmitoyl pentapeptide-4, nonapeptide-1, hexapeptide-2, acetyl hexapeptide-8, acetyl octapeptide-3, acetyl hexapeptide-1 o acetyl tetrapeptide-2, and combinations thereof.

3. The cosmetic composition according to claim 2, wherein the anti-aging or antioxidant peptide is acetyl tetrapeptide-2.

4. The cosmetic composition according to any one of the previous claims, wherein the anti-aging or antioxidant peptide is in an amount of between 0,0005% and 0,005% by weight with respect to the total weight of the composition.

5. The cosmetic composition according to any one of the previous claims, wherein the growth factor is selected from the group consisting of epidermal growth factor, insulin-like growth factor 1, transforming growth factor beta-2, Nicotiana Benthamiana Hexapeptide-40 sh-Polypeptide-76, sh-Polypeptide-1, sh-Polypeptide-9, sh-Polypeptide-11, Oligopeptide-4, Oligopeptide-2, Oligopeptide-1, and combinations thereof.

6. The cosmetic composition according to claim 5, wherein the growth factor is Oligopeptide-1, epidermal growth factor, and/or combinations thereof.

7. The cosmetic composition according to any one of the previous claims, wherein the growth factor is in an amount of between 0,000001% and 0,000003% by weight with respect to the total weight of the composition.

8. The cosmetic composition according to any one of the previous claims, wherein the phospholipids are derived from lecithin.

9. The cosmetic composition according to any one of the previous claims, wherein the phospholipids are in an amount of between 0,1% and 2% by weight with respect to the total amount of the composition.

10. The cosmetic composition according to any one of the previous claims, wherein the encapsulating agent is a liposome.

11. The cosmetic composition according to claim 10, wherein the liposome comprises one lipidic bilayer.

12. The cosmetic composition according to any one of the previous claims, further comprising an emollient.

13. The cosmetic composition according to claim 12, wherein the emollient is selected from the group consisting of squalene, sweet almond oil and combinations thereof.

14. Method for rejuvenating the skin, **characterized in that** it comprises the topical application of the cosmetic composition according to any of the claims 1 to 13.

15. Use of the cosmetic composition according to any of the claims 1 to 13 for rejuvenating the skin, by means of its topical application.
